# FASCICULE DE BREVET EUROPEEN

(11) **EP 0 519 050 B1**
(45) Date de publication et mention de la délivrance du brevet: **15.07.1998**
(21) Numéro de dépôt: 92903478.3
(22) Date de dépôt: 26.12.1991
(51) Int. Cl.: B01J 19/24, B01J 19/28, C07C 17/25, C07C 21/06, C10G 9/16

(54) **PROCEDE ET DISPOSITIF DE FABRICATION DE PRODUITS CHIMIQUES**
VERFAHREN UND VORRICHTUNG ZUR HERSTELLUNG VON CHEMISCHEN PRODUKTEN
METHOD AND DEVICE FOR MANUFACTURING CHEMICALS

(30) Priorité: 28.12.1990 FR 9016627
(43) Date de publication de la demande: 23.12.1992
(73) Titulaire: NAPHTACHIMIE S.A., F-92400 Courbevoie (FR)
(72) Inventeur: BELLET, Serge, F-13200 Châteauneuf-les-Martigues (FR); LOUBLIER, Marc, F-13960 Sausset-les-Pins (FR); MARGAIL, Guy, F-13500 Martigues (FR)
(74) Mandataire: Somnier, Jean-Louis
(86) Numéro de dépôt international: FR9101074
(87) Numéro de publication internationale: WO9211931

(56) Documents cités:
- FR-A- 2 221 426
- FR-A- 2 600 665
- US-A- 3 183 967
- US-A- 3 819 740
- US-A- 4 271 007

## Description

La présente invention se rapporte à un procédé de fabrication d'un ou plusieurs produits chimiques comportant une réaction chimique réalisée à l'intérieur d'un tube. La présente invention a également pour objet un dispositif constitué par un four destiné à la mise en oeuvre de ce procédé.

Il est connu de réaliser des réactions chimiques à haute température par des procédés qui consistent à faire circuler les réactifs dans un tube maintenu à haute température et qui est disposé à l'intérieur d'une zone de radiation d'un four. Plus particulièrement, pour fabriquer des oléfines, notamment de l'éthylène, du propylène ou de l'isobutène, il est connu de réaliser une réaction de craquage à la vapeur d'eau, d'hydrocarbures liquides ou gazeux dans des fours dont la température de sortie est généralement comprise entre 750°C et 880°C. Dans ce procédé connu sous le nom de craquage ou de pyrolise à la vapeur d'eau ou encore sous le nom de vapocraquage, on fait passer à travers la zone de radiation d'un four un mélange d'hydrocarbures et de vapeur d'eau circulant dans un tube de craquage disposé sous la forme d'un serpentin à l'intérieur de ce four. Toutes ces réactions chimiques sont le plus souvent effectuées dans des fours modernes comprenant en général un dispositif de chauffe constitué de brûleurs disposés sur la sole et/ou sur les murs internes du four. La puissance thermique de chauffe de ces brûleurs est avantageusement répartie le long du tube de telle sorte que les réactifs de la réaction soient soumis à une température qui augmente le long du tube depuis la température d'entrée de la zone de radiation jusqu'à la température de sortie de cette zone.

Lorsque le tube est maintenu à haute température, il est important de bien contrôler la température de sa surface externe, dite température de peau. En effet, compte tenu des contraintes thermiques du tube et du fait que l'on désire généralement éviter un vieillissement prématuré du tube, on règle la puissance thermique de chauffe des brûleurs de telle sorte que la température de peau mesurée à n'importe quel endroit du tube soit toujours inférieure à une température de peau limite.

Celle-ci est une caractéristique technique du tube et dépend largement de la durée de vie souhaitée pour le tube ainsi que de la nature du métal ou de l'alliage constituant le tube. Lorsqu'on réalise une réaction de craquage à la vapeur d'eau d'hydrocarbures, la température de peau limite est en général comprise entre 1050°C et 1110°C.

La plupart des réactions chimiques réalisées dans un tube présentent l'inconvénient majeur de générer des sous-produits qui se déposent sur la paroi interne du tube. Ainsi en réalisant un procédé de vapocraquage d'hydrocarbures on observe, notamment lorsque la température de craquage est élevée et/ou lorsque le tube de craquage est alimenté suivant un débit important, qu'il se forme une couche de coke à l'intérieur du tube de craquage et ce, malgré un écoulement turbulent du mélange d'hydrocarbures et de vapeur d'eau. Les dépôts de sous-produits sont particulièrement gênants, car ils limitent sensiblement les transferts thermiques à travers la paroi du tube. Aussi afin de maintenir une température de réaction constante, on observe qu'il est nécessaire d'augmenter la puissance thermique délivrée par les brûleurs au fur et à mesure de l'augmentation de l'épaisseur de la couche de sous-produits, ce qui se traduit par une augmentation de la consommation énergétique du four. D'autre part consécutivement à l'augmentation de la puissance thermique délivrée par les brûleurs on constate une augmentation de la température de peau du tube.

Ce phénomène est particulièrement gênant, car plus les dépôts de sous-produits augmentent, plus il est difficile, voire impossible de maintenir une température de peau inférieure à la température de peau limite si l'on désire réaliser la réaction avec une production et un rendement satisfaisants. Dans ces conditions, il est nécessaire d'arrêter régulièrement la réaction afin de procéder à une élimination des dépôts de sous-produits. Dans le cas particulier d'une réaction de vapocraquage, cette élimination s'effectue par une opération de décokage du tube de craquage. Cette opération consiste à faire circuler un mélange d'air et de vapeur d'eau à l'intérieur du tube de craquage qui est maintenu à un température suffisante pour pouvoir brûler et éliminer tout le coke présent. Dans la pratique, on observe qu'une opération de décokage est relativement longue, généralement d'une durée totale voisine de 24 heures et qu'il est souhaitable de décoker un tube selon une fréquence élevée, le plus souvent voisine de 2 à 3 mois.

Compte tenu du fait que les réactions chimiques sont généralement effectuées dans des installations ayant une grande taille, les arrêts nécessaires pour éliminer les couches de sous-produits à l'intérieur du tube conduisent à un manque important de production. Ainsi depuis plusieurs années de nombreuses études ont été menées aussi bien en laboratoire qu'au stade industriel dans le but de trouver un procédé permettant de limiter les dépôts de sous-produits sur la paroi interne du tube.

Il est connu selon le brevet US 4 368 677 un procédé qui permet de prévenir le dépôt de sous-produits d'une réaction de combustion, sur la paroi externe d'un échangeur de chaleur tubulaire disposé dans une chaudière et à l'intérieur duquel il y a une circulation d'eau. Selon ce procédé on utilise plusieurs courants gazeux pulsés qui délivrent à l'intérieur de la chaudière, un gaz chaud, de la chaleur et des ondes sonores. En particulier, on utilise un premier courant gazeux pulsé afin de produire un combustible constitué de particules ayant une taille uniforme. Par ailleurs, on dirige un second courant gazeux sur la paroi externe de l'échangeur de préférence parallèlement aux tubes constituant l'échangeur, afin d'éviter leur vibration. En conséquence, ce procédé ne peut pas être utilisé pour limiter les dépôts de sous-produits sur la paroi interne d'un tube dans lequel on réalise une réaction chimique.

Le document US-A-3819740 décrit par ailleurs un procédé de craquage thermique d'hydrocarbures dans lequel on applique à un mélange d'hydrocarbures et de vapeur d'eau surchauffée circulant dans une chambre réactionnelle, des vibrations ultrasoniques de fréquence 1 - 800 kHz ; l'application des vibrations ultrasoniques sert à diminuer la formation de coke et à augmenter le rendement de la réaction.

Il a été maintenant trouvé un procédé de fabrication qui permet notamment de réaliser une réaction chimique à l'intérieur d'un tube disposé dans l'enceinte de radiation d'un four, tout en évitant les inconvénients cités auparavant. En particulier, il permet de limiter les dépôts de sous-produits sur la paroi interne du tube, notamment sans être obligé d'arrêter la réaction chimique et sans modifier son rendement et/ou sa production. Par ailleurs, le procédé de l'invention peut être facilement adapté aux installations industrielles déjà existantes.

La présente invention concerne tout d'abord un procéde de fabrication ou de plusieurs produits chimiques dans lequel on réalise une réaction chimique en faisant circuler un ou plusieurs réactifs à l'intérieur d'un tube, dans lequel 'au moins une partie de tube est soumis à une vibration afin de limiter les dépôts de sous-produits de la réaction sur la paroi interne du tube, caractérisé en ce que ladite vibration est transversale, et en ce que ledit tube est disposé dans une zone de radiation d'un four.

Selon le procédé de l'invention, il est essentiel de soumettre le tube à une vibration si l'on désire limiter les dépôts de sous-produits sur sa paroi interne. Pour cela, la vibration du tube a le plus souvent une fréquence comprise entre 50 et 2000 Hertz, de préférence comprise entre 100 et 1000 Hertz.

La vibration transversale du tube peut être telle qu'au moins un point de ce tube vibre avec une amplitude qui représente le déplacement de ce point entre ses deux positions extrêmes, supérieure à 10⁻⁶ de préférence à 10⁻⁴ fois le diamètre interne du tube. Toutefois, on préfère avoir une amplitude de vibration de tous les points du tube pas trop importante afin d'éviter une diminution prématurée des propriétés mécaniques du tube qui peut entraîner sa rupture et/ou la rupture de ses fixations. Par ailleurs, la vibration transversale du tube peut s'effectuer selon l'une de ses fréquences propres. Dans ce cas, la vibration transversale du tube est caractérisée par une déformation comportant des noeuds de vibration dont l'amplitude de déplacement est nulle et des ventres de vibration dont l'amplitude est maximale. La position des noeuds et des ventres de vibration peut être changée plusieurs fois au cours du procédé, en choisissant des fréquences propres de vibration différentes.

Selon le procédé de l'invention, la limitation des dépôts de sous-produits sur la paroi interne du tube peut consister à éviter totalement ou partiellement les dépôts ou encore à éliminer totalement ou partiellement les dépôts déjà existants. La vibration du tube peut être effectuée dans le temps de façon isolée ou de façon intermittente. Dans ce cas, le procédé est plutôt adapté à une élimination des dépôts existants. La vibration du tube peut également être effectuée d'une façon continue dans ce cas, le procédé est plutôt adapté pour éviter les dépôts.

La vibration du tube peut être provoquée par tout moyen. En particulier, elle peut être provoquée par une source de vibrations mécaniques. D'autre part, compte tenu qu'en général la zone de radiation ne fonctionne pas sous vide et qu'elle contient un mélange gazeux, la vibration du tube peut également être provoquée par une onde de pression extérieure au tube. Cette onde peut se propager selon une direction quelconque. En particulier, elle peut se propager selon une direction parallèle ou sensiblement parallèle au tube ou selon une direction perpendiculaire ou sensiblement perpendiculaire au tube. Par ailleurs, l'onde de pression peut être une onde stationnaire de pression établie à l'extérieur du tube, dans la zone de radiation. Cette onde stationnaire est caractérisée par le fait qu'il se forme dans la zone de radiation des ventres de pression et des noeuds de pression dont les dispositions dépendent de la longueur de l'onde stationnaire. D'une manière avantageuse, lorsque la direction de l'onde stationnaire est perpendiculaire a la direction du tube, on choisit une longueur d'onde de façon à ce qu'un ventre de pression, soit à proximité du tube, afin d'augmenter sensiblement l'efficacité du procédé.

Etant donné que l'enceinte de radiation du four est munie de brûleurs, le mélange gazeux contenu dans cette enceinte provient en partie des gaz de la combustion réalisée dans les différents brûleurs. D'autre part, selon le procédé de l'invention, pour certaines dispositions des brûleurs sur les parois de l'enceinte de radiation et/ou pour certaines compositions du combustible alimentant les brûleurs, les vibrations des flammes de ces brûleurs peuvent générer des ondes de pression suffisantes pour faire vibrer le tube de façon à limiter les dépôts de sous-produits.

La présente invention repose sur la découverte étonnante qu'une vibration du tube permet de limiter considérablement les dépôts de sous-produits sur sa paroi interne, sans pour autant modifier les conditions de la réaction chimique et notamment son rendement. Par ailleurs, on a constaté avec surprise que la vibration du tube peut être réalisée sans pratiquement aucun vieillissement prématuré de celui-ci et en particulier aucune diminution prématurée de ses propriétés mécaniques. L'avantage du procédé réside essentiellement en une réduction importante de la fréquence des opérations d'élimination des dépôts de sous-produits par des moyens différents de ceux mis en oeuvre dans les procédés connus.

Grâce au procédé de l'invention, on peut réaliser toute réaction chimique habituellement réalisée dans un tube. La réaction peut être effectuée en phase liquide ou en phase gazeuxe et les réactifs peuvent circuler à l'intérieur du tube selon un écoulement laminaire ou turbulent et le plus souvent continu. La température de réaction peut être notamment comprise entre 100 et 900°C et en particulier entre 500 et 850°C.

La réaction chimique peut être une réaction de craquage thermique de différents produits organiques, notamment de dichloro-1,2 éthane en vue d'obtenir du chlorure de vinyle.

Le procédé est particulièrement adapté au craquage d'un mélange d'eau et d'hydrocarbures, dite réaction de vapocraquage, réalisée dans un tube de craquage disposé dans la zone de radiation d'un four de craquage. Dans ce cas, pour réaliser le procédé la température de craquage augmente le long du tube de craquage, depuis l'entrée jusqu'à la sortie de la zone de radiation du four, c'est à dire dans le sens d'écoulement du mélange réactionnel. En particulier, la température de craquage du mélange d'hydrocarbures et de vapeur d'eau est à l'entrée de la zone de radiation du four comprise entre 500°C et 700°C de préférence comprise entre 550°C et 660°C elle est à la sortie de cette zone comprise entre 800°C et 880°C, de préférence comprise entre 810°C et 860°C, l'augmentation de la température de craquage le long du tube peut être quelconque, uniforme ou telle que décrite dans les brevets français FR-A-2600665 et FR-A-2600667. Par ailleurs, le mélange d'hydrocarbures et de vapeur d'eau est, généralement soumis à un préchauffage avant son entrée dans la zone de radiation du four, ce préchauffage pouvant être réalisé par tout moyen connu, notamment dans une zone de chauffage par convection du four. Le temps de séjour moyen du mélange d'hydrocarbures et de vapeur d'eau circulant dans le tube entre l'entrée et la sortie de la zone de radiation du four est généralement compris entre 300 et 1800 millisecondes, de préférence entre 400 et 1400 millisecondes. La composition du mélange d'hydrocarbures et de vapeur d'eau, est telle eue le rapport pondéral de la quantité d'hydrocarbures à la quantité de vapeur d'eau est compris entre 1 et 10, de préférence compris entre 2 et 6.

Lors du procédé de craquage à la vapeur d'eau, on peut mettre en oeuvre aussi bien des hydrocarbures liquides ou gazeux. Les hydrocarbures liquides peuvent être choisis parmi le naphta constitué d'hydrocarbures comportant environ de 5 à 10 atomes de carbones. Les essences légères constituées d'hydrocarbures comportant environ de 5 à 6 atomes de carbone, le gas oil constitué d'hydrocarbures comportant environ de 8 à 15 atomes de carbone, ainsi que leurs mélanges. Ils peuvent en oeutre être utilisés en mélange avec des hydrocarbures saturés et insaturés comportant de 3 à 6 atomes de carbone. Les hydrocarbures gazeux peuvent être des alcanes comportant de 2 à 4 atomes de carbone et/ou de méthane et/ou des alcanes comportant de 5 ou 6 atomes de carbone. On peut en particulier, mettre en oeuvre dans le procédé de craquage à la vapeur d'eau du gaz naturel, du gaz de pétrole liquéfié également appelé LPG ou de l'éthane, produit secondaire issu du vaprocraquage d'hydrocarbures liquides tels que le naphta ou le gasoil.

La présente invention concerne églament un dispositif permettant la mise en oeuvre du procédé décrit précédemment. Ce dispositif est constitué d'un four comprenant une enceinte thermique de radiation munie de moyens de chauffe constitués par des brûleurs, et à travers laquelle passe au moins un tube où circule un ou plusieurs réactif(s), caractérisé en ce qu'il comprend une source de vibration mécanique reliée au tube par l'intermédiaire d'une liaison mécanique, ou une source d'onde de pression sonore établie extérieurement au tube et dirigée dans une direction sensiblement perpendiculaire au tube, de façon à provoquer une vibration transversale d'au moins une partie du tube afin de limiter sur sa paroi interne, les dépôts de sous-produits obtenus lors d'une réaction chimique effectuée à l'intérieur du tube.

Le dispositif de l'invention doit être constitué d'un four comprenant au moins un tube disposé dans son enceinte thermique de radiation. Le tube a généralement un diamètre interne moyen allant de 20 à 200 mm, de préférence de 30 à 150 mm, une épaisseur allant de 3 à 15 mm et est le plus souvent constitué d'une section droite ou de plusieurs sections droites reliées entre elles, notamment par des coudes. Le four est adapté à la réaction chimique réalisée. En particulier, lorsque le procédé est utilisé pour effectuer une réaction de vapocraquage, le four doit être un four de vapocraquage qui comprend une enceinte thermique de radiation à travers laquelle, passe au moins un tube de craquage disposé sous la forme d'un serpentin horizontal ou vertical. Ce tube de craquage présente généralement un rapport entre la longueur et le diamètre moyen interne compris entre 30 et 1500 et de préférence compris entre 300 et 1000. En particulier, le diamètre moyen interne du tube de craquage est de préférence égal ou supérieur à 100 mm, de telle sorte que le temps de séjour moyen du mélange réactionnel dans le tube de craquage puisse être relativement important et que les pertes de charge du mélange circulant dans le tube de craquage puissent être faibles. Toutefois, le diamètre moyen interne et la longueur de tube doivent rester dans des domaines de valeurs compatibles avec les contraintes mécaniques et thermiques, auxquelles sont soumis les matériaux constituant le tube de craquage. En particulier, le diamètre moyen interne du tube de craquage ne peut excéder 250 mm environ. D'une manière avantageuse, le tube de craquage peut avoir un diamètre interne variable sur sa longueur comme décrit dans les brevets français FR-A-2600667 et FR-A-2600641. Dans la pratique, le tube de craquage est disposé sous la forme d'un serpentin constitué d'une succession de sections droites reliées entre elles par des coudes.

Le moyen d'excitation, du dispositif de l'invention peut être un générateur de vibrations mécaniques, disposé à l'intérieur ou à l'extérieur de la zone de radiation du four et relié au tube par l'intermédiaire d'une liaison mécanique. La source de vibrations peut être une machine vibrante.

Le moyen d'excitation peut également être un générateur d'ondes de pression et en particulier d'ondes sonores. Dans le cas où on génère des ondes de pression stationnaires, les parois de la zone de radiation doivent permettre la réflexion des ondes. Le générateur d'ondes de pression peut être une membrane vibrante, telle que celle d'un haut parleur, une sirène ou encore une corne de brume. Le moyen d'excitation peut encore être constitué d'un ou plusieurs brûleurs, chaque brûleur étant équipé d'un moyen destiné à générer des pulsations du débit de son combustible d'alimentation. Ce moyen générateur de pulsations peut être un obturateur périodique, de la sortie de l'alimentation en combustible actionné à l'aide d'un pot vibrant.

Les figures (1) et (2) représentent schématiquement un four de vapocraquage selon deux coupes différentes.

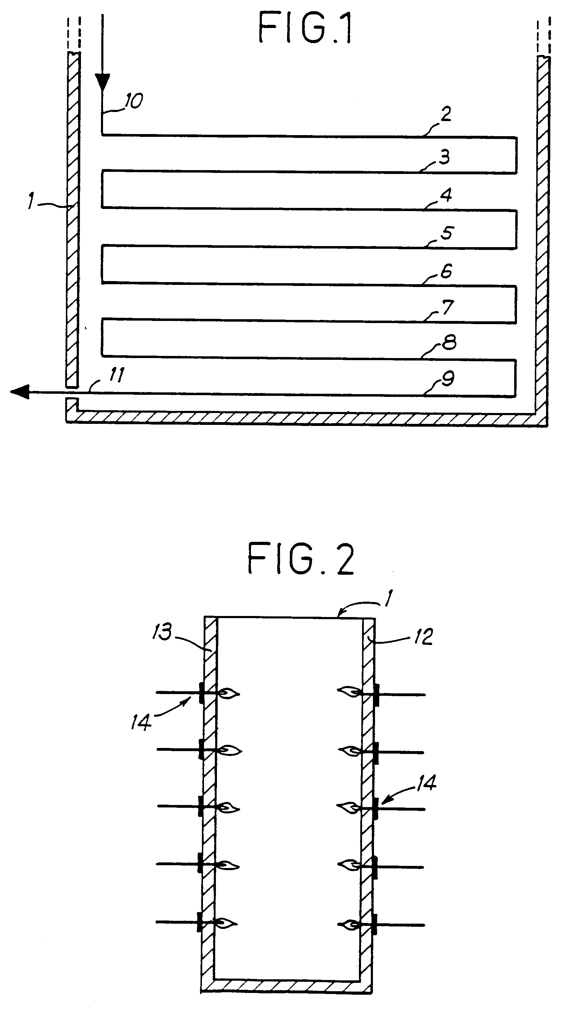

La figure 1 illustre schématiquement selon une coupe longitudinale un four horizontal de vaprocraquage, comprenant une enceinte thermique de radiation (1), à travers laquelle passe un tube de craquage disposé sous la forme d'un serpentin constitué de huit sections droites horizontales reliées entre elles par des coudes, les sections (2) à (9) ont un diamètre interne moyen de 108 mm. L'entrée et la sortie du tube de craquage dans l'enceinte thermique de radiation sont respectivement en (10) et (11). Ce four de vapocraquage comprend une enceinte thermique de radiation munie de moyens de chauffe constitués de brûleurs, disposés en rangées sur les murs de l'enceinte. La disposition, le règlage et/ou la taille des brûleurs dans l'enceinte thermique, sont tels que les vibrations des flammes des brûleurs produisent un système d'ondes de pression à l'intérieur de l'enceinte thermique de radiation.

La figure 2 représente schématiquement l'enceinte de radiation (1) d'un four de vapocraquage, selon une coupe transversale contenant des brûleurs. Les murs (12) et (13) sont chacun équipés de 5 rangées de brûleurs (14). Seulement un brûleur par rangée est représenté sur la figure.

L'exemple suivant illustre la présente invention.

### Exemple 1

On opère dans un four de vapocraquage, tel que représenté schématiquement à la figure 1, qui comprend une enceinte thermique de radiation (1) en briquetage, constitué par un parallélipipède rectangle dont les dimensions internes sont de 9,75 m pour la longueur, de 1,70 m pour la largeur de 4,85 m pour la hauteur. Dans l'enceinte (1), on place un tube de craquage en acier réfractaire à base de nickel et de chrome ayant un diamètre interne de 108 mm, une épaisseur de 8 mm et compte tenu de la capacité de l'enceinte et des contraintes thermiques du four, une longueur totale de 80 mètres, comprise entre l'entrée et la sortie de l'enceinte. Ce tube de craquage est disposé sous la forme d'un serpentin, comprenant huit sections droites horizontales d'égale longueur chacune reliées entre elles par des coudes. Le diamètre interne de ces sections droites est constant et égal à 108 mm.

L'enceinte thermique de radiation du four de vapocraquage est munie de brûleurs disposés sur les murs de l'enceinte suivant cinq rangées horizontales situées à égale distance les unes des autres. La puissance thermiqmue de l'ensemble de ces brûleurs est répartie de façon homogène entre ces cinq rangées.

Tous les brûleurs sont alimentés, d'une part, suivant un débit sensiblement constant de 1750 kg/heure combustible constitué d'un mélange gazeux comprenant en volume 90 % de méthane, et 10 % d'hydrogène et, d'autre part, d'air préchauffé à 100°C en quantité suffisante pour assurer une bonne combustion du mélange gazeux.

Dans ce tube de craquage, on fait circuler un mélange d'hydrocarbures liquides et de vapeur d'eau. Les hydrocarbures liquides sont constitués par un naphta de densité 0,717, ayant un intervalle de distillation ASTM 44/165°C et des teneurs pondérales de 32,74 % en paraffines linéaires, de 30,95 % en paraffines ramifiées, de 28,80 % en composés cyclaniques et de 7,51 % en composés aromatiques. La composition du mélange de naphta et de vapeur d'eau mise en oeuvre est telle, que le rapport pondéral de la quantité de naphta à la quantité de vapeur d'eau est de 4. On introduit ainsi dans le tube de craquage le naphta suivant un débit de 3700 kg/h et la vapeur d'eau suivant un débit de 925 kg/h.

La température de craquage du mélange du naphta et de la vapeur d'eau s'élève de 570°C à l'entrée de la zone de radiation du four jusqu'à 810°C à la sortie de cette zone. La pression du mélange est à la sortie du four de 170 kPa. Le temps de séjour moyen de mélange de naphta et de vapeur d'eau circulant dans le tube de craquage entre l'entrée et la sortie de la zone de radiation du four est de 900 millisecondes.

Dans ces conditions, on produit par heure 752 kg d'éthylène, 598 kg de propylène, 102 kg d'isobutène, 157 kg de butadiène et 186 kg d'éthane et on mesure une température de peau maximum de 990°C.

Durant deux jours, on modifie l'alimentation des brûleurs de façon à les alimenter par 1750 kg/heure d'un nouveau mélange gazeux comprenant en volume 75 % de méthane et 25 % d'hydrogène. Dans ces nouvelles conditions, la production et le rendement du four ne sont pas modifiés et on observe une vibration du tube de craquage provenant des ondes générées par les vibrations des flammes des brûleurs. Cette vibration du tube de craquage élimine une partie du coke déposé auparavant sur sa paroi interne ce qui se manifeste, à l'issue des deux jours, par une nouvelles température de peau maximum inférieure à la précédente et voisine de 950°C.

## Revendications

1. Procédé de fabrication d'un ou plusieurs produits chimiques dans lequel on réalise une réaction chimique en faisant circuler un ou plusieurs réactifs à l'intérieur d'un tube, dans lequel on provoque une vibration d'au moins une partie du tube, de façon à limiter des dépôts de sous-produits de la réaction sur la paroi interne du tube, caractérisé en ce que ladite vibration est transversale, et en ce que ledit tube est disposé dans une zone de radiation d'un four.

2. Procédé selon la revendication 1 dans lequel on provoque ladite vibration transversale à l'aide d'une source de vibration mécanique reliée au tube par l'intermédiaire d'une liaison mécanique, ou à l'aide d'une source d'onde de pression sonore établie extérieurement au tube.

3. Procédé selon la revendication 1 ou 2, caractérisé en ce que la vibration d'au moins une partie du tube est réalisée à une fréquence comprise entre 50 et 2000 Hertz.

4. Procédé selon l'une quelconque des revendications 1 à 3, caractérisé en ce que la vibration transversale est une déformation du tube comportant des noeuds et des ventres dont la position est changée plusieurs fois au cours du procédé.

5. Procédé selon la revendication 2, caractérisé en ce que la source de vibration mécanique est disposée à l'extérieur de la zone de radiation du four.

6. Procédé selon revendication 2, caractérisé en ce que l'onde de pression est propagée selon une direction sensiblement perpendiculaire au tube.

7. Procédé selon la revendication 2 ou 6 caractérisé en ce que l'onde de pression est générée par la vibration des flammes de brûleurs disposés dans la zone de radiation du four.

8. Procédé selon la revendication 7, caractérisé en ce qu'on alimente les brûleurs à l'aide d'un combustible capable de faire vibrer les flammes desdits brûleurs.

9. Procédé selon la revendication 7 caractérisé en ce que la vibration des flammes des brûleurs est obtenue par pulsation du débit du combustible d'alimentation des brûleurs.

10. Procédé selon la revendication 9 caractérisé en ce qu'on obture périodiquement la sortie d'alimentation en combustible des brûleurs.

11. Procédé selon l'une quelconque des revendications 1 à 10 caractérisé en ce que la vibration d'au moins une partie du tube est réalisée au cours du temps d'une façon isolée ou intermittente.

12. Procédé selon l'une quelconque des revendications 1 à 10, caractérisé en ce que la vibration d'au moins une partie du tube est réalisée d'une façon continue au cours du temps.

13. Procédé selon l'une quelconque des revendications 1 à 12, caractérisé en ce que la réaction chimique est une réaction de craquage du dichloro-1,2 éthane.

14. Procédé selon l'une quelconque des revendications 1 à 12 caractérisé en ce que la réaction chimique est une réaction de craquage d'un mélange d'hydrocarbures et de vapeur d'eau.

15. Procédé selon l'une quelconque des revendications 1 à 14, caractérisé en ce que la vibration transversale du tube est réalisée de telle sorte qu'au moins un point du tube vibre avec une amplitude supérieure à 10⁻⁴ fois le diamètre interne du tube.

16. Procédé selon l'une quelconque des revendications 1 à 15, caractérisé en ce que la vibration transversale du tube est réalisée à une fréquence comprise entre 100 et 1000 Hz.

17. Procédé selon l'une quelconque des revendications 1 à 16, caractérisé en ce que la vibration transversale du tube est réalisée à l'une des fréquences propres du tube.

18. Dispositif pour la fabrication d'un ou plusieurs produit(s) chimique(s) constitué d'un four comprenant une enceinte thermique de radiation munie de moyens de chauffe constitués par des brûleurs et à travers laquelle passe au moins un tube où circule un ou plusieurs réactif(s), caractérisé en ce qu'il comprend une source de vibration mécanique reliée au tube par l'intermédiaire d'une liaison mécanique, ou une source d'onde de pression sonore établie extérieurement au tube et dirigée dans une direction sensiblement perpendiculaire au tube, de façon à provoquer une vibration transversale d'au moins une partie du tube et à limiter ainsi des dépôts sur la paroi interne du tube.

19. Dispositif selon la revendication 18, caractérisé en ce que le tube est un tube de craquage ayant un diamètre moyen interne allant de 100 à 250 mm.

20. Dispositif selon la revendication 18 ou 19, caractérisé en ce que la source de vibration mécanique est disposée à l'extérieur de l'enceinte thermique de radiation .

21. Dispositif selon l'une quelconque des revendications 18 à 20 caractérisé en ce que la source de vibration mécanique est une machine vibrante.

22. Dispositif selon la revendication 18 ou 19 caractérisé en ce que l'onde de pression est générée par une membrane vibrante.

23. Dispositif selon la revendication 18 ou 19 caractérisé en ce que l'onde de pression est générée par un ou plusieurs brûleurs de l'enceinte thermique de radiation.

24. Dispositif selon la revendication 23 caractérisé en ce que le ou les brûleurs sont équipés d'un moyen destiné à générer des pulsations du débit du combustible d'alimentation des brûleurs.

25. Dispositif selon la revendication 24 caractérisé en ce que le moyen générateur des pulsations est un obturateur périodique de la sortie d'alimentation en combustible des brûleurs.

26. Dispositif selon l'une quelconque des revendications 18 à 25, caractérisé en ce que le four est un four de vapocraquage destiné à craquer un mélange d'hydrocarbures et de vapeur d'eau.

27. Dispositif selon l'une quelconque des revendications 18 à 25, caractérisé en ce que le four est un four de craquage thermique du dichloro-1, 2 éthane.

## Claims

1. A method of manufacturing one or more chemical products in which a chemical reaction is performed by causing one or more reagents to flow inside a tube in which at least a portion of the tube is set into transverse vibration for the purpose of limiting the deposition of reaction by-products on the inside wall of the tube, the method being characterized in that said vibration is transverse, and in that said tube is disposed in a radiation zone of a furnace.

2. A method according to claim 1, in which said transverse vibration is caused by means of a mechanical vibration source connected to the tube by means of a mechanical link, or by means of a pressure soundwave established outside the tube.

3. A method according to claim 1 or 2, characterized in that at least a portion of the tube is vibrated at a frequency lying in the range 50 Hz to 2000 Hz.

4. A method according to any one of claims 1 to 3, characterized in that the transverse vibration is a deformation of the tube that includes nodes and antinodes whose positions are changed several times during the method.

5. A method according to claim 2, characterized in that the mechanical vibration source is disposed outside the radiation zone of the furnace.

6. A method according to claim 2, characterized in that the pressure wave is propagated in a direction substantially perpendicular to the tube.

7. A method according to claim 2 or 6, characterized in that the pressure wave is generated by the vibration of the flames of burners disposed in the radiation zone of the furnace.

8. A method according to claim 7, characterized in that the burners are fed by means of a fuel capable of causing the flames of said burners to vibrate.

9. A method according to claim 7, characterized in that vibration of the flames of the burners is obtained by pulses in the flow of fuel feeding the burners.

10. A method according to claim 9, characterized in that the fuel feed outlet of the burners is periodically closed.

11. A method according to any one of claims 1 to 10, characterized in that at least a portion of the tube is vibrated at isolated moments in time or intermittently.

12. A method according to any one of claims 1 to 10, characterized in that at least a portion of the tube is vibrated continuously over time.

13. A method according to any one of claims 1 to 12, characterized in that the chemical reaction is a cracking reaction of dicholoro-1,2 ethane.

14. A method according to any one of claims 1 to 12, characterized in that the chemical reaction is a cracking reaction performed on a mixture of hydrocarbons and steam.

15. A method according to any one of claims 1 to 14, characterized in that the tube is vibrated transversely so that at least one point of the tube vibrates with an amplitude greater than 10⁻⁴ times the inside diameter of the tube.

16. A method according to any one of claims 1 to 15, characterized in that the tube is vibrated transversely at a frequency lying in the range 100 Hz to 1000 Hz.

17. A method according to any one of claims 1 to 16, characterized in that the tube is vibrated transversely at a resonant frequency of the tube.

18. Apparatus for manufacturing one or more chemical products, the apparatus being constituted by a furnace comprising a radiation thermal enclosure provided with heater means constituted by burners, the enclosure having at least one tube passing therethrough where one or more reagents flow, the apparatus being characterized in that it comprises a mechanical vibration source connected to the tube by means of a mechanical link, or by means of a pressure soundwave established outside the tube and directed in a direction substantially perpendicular to the tube so as to cause transverse vibration in at least a portion of the tube and thus limit deposits on the inside wall of the tube.

19. Apparatus according to claim 18, characterized in that the tube is a cracking tube have a mean inside diameter lying in the range 100 mm to 250 mm.

20. Apparatus according to claim 18 or 19, characterized in that the mechanical vibration source is disposed outside the radiation thermal enclosure.

21. Apparatus according to any one of claims 18 to 20, characterized in that the mechanical vibration source is a vibrating machine.

22. Apparatus according to claim 18 or 19, characterized in that the pressure wave is generated by a vibrating membrane.

23. Apparatus according to claim 18 or 19, characterized in that the pressure wave is generated by one or more burners of the radiation thermal chamber.

24. Apparatus according to claim 23, characterized in that the burner(s) is/are fitted with means designed to generate pulses in the flow of fuel feeding the burners.

25. Apparatus according to claim 24, characterized in that the pulse generator means is a periodically-operating shutter, serving to shut the fuel feed outlet of the burners.

26. Apparatus according to any one of claims 18 to 25, characterized in that the furnace is a steam cracking furnace designed to crack a mixture of hydrocarbons and steam.

27. Apparatus according to any one of claims 18 to 25, characterized in that the furnace is a thermal cracking furnace for dicholoro-1,2 ethane.

## Patentansprüche

1. Verfahren zur Herstellung eines oder mehrerer chemischer Produkte, bei dem man eine chemische Reaktion durchführt, indem man einen oder mehrere der Reaktanten im Innern eines Rohrs zirkulieren läßt, wobei man in dem Rohr eine Vibration mindestens eines Teils des Rohrs hervorruft, um Abscheidungen von Nebenprodukten der Reaktion an der Innenwand des Rohrs zu beschränken, dadurch gekennzeichnet, daß die Vibration transversal verläuft und daß das Rohr in einer Strahlungszone eines Ofens angeordnet ist.

2. Verfahren nach Anspruch 1, wobei man die transversale Vibration mittels einer mechanischen Vibrationsquelle, die über eine mechanische Verbindung mit dem Rohr verbunden ist, oder mittels einer von außen an das Rohr angelegten Quelle für Schalldruckwellen ausübt.

3. Verfahren nach Anspruch 1 oder 2, dadurch gekennzeichnet, daß die Vibration von mindestens einem Teil des Rohrs eine Frequenz von 50 bis 2000 Hz aufweist.

4. Verfahren nach einem der Ansprüche 1 bis 3, dadurch gekennzeichnet, daß es sich bei der transversalen Vibration um eine Deformation des Rohrs handelt, die Knoten und Bäuche aufweist, deren Position sich im Verlauf des Verfahrens mehrmals ändert.

5. Verfahren nach Anspruch 2, dadurch gekennzeichnet, daß die mechanische Vibrationsquelle außerhalb der Strahlungszone des Ofens angeordnet ist.

6. Verfahren nach Anspruch 2, dadurch gekennzeichnet, daß die Druckwelle sich in einer Richtung, die im wesentlichen senkrecht zum Rohr verläuft, ausbreitet.

7. Verfahren nach Anspruch 2 bis 6, dadurch gekennzeichnet, daß die Druckwelle durch die Vibration von Brennerflammen, die in der Strahlungszone des Ofens angeordnet sind, erzeugt wird.

8. Verfahren nach Anspruch 7, dadurch gekennzeichnet, daß man die Brenner mit einem Brennstoff versorgt, der dazu befähigt ist, die Flammen der Brenner zum Vibrieren zu bringen.

9. Verfahren nach Anspruch 7, dadurch gekennzeichnet, daß die Vibration der Flammen der Brenner durch einen pulsierenden Ausstoß des Brennstoffs zur Versorgung der Brenner erreicht wird.

10. Verfahren nach Anspruch 9, dadurch gekennzeichnet, daß man periodisch den Ausgang zur Versorgung der Brenner mit dem Brennstoff verschließt.

11. Verfahren nach einem der Ansprüche 1 bis 10, dadurch gekennzeichnet, daß die Vibration von mindestens einem Teil des Rohrs im Laufe der Zeit in isolierter oder intermittierender Weise erfolgt.

12. Verfahren nach einem der Ansprüche 1 bis 10, dadurch gekennzeichnet, daß die Vibration von mindestens einem Teil des Rohrs im Laufe der Zeit in kontinuierlicher Weise erfolgt.

13. Verfahren nach einem der Ansprüche 1 bis 12, dadurch gekennzeichnet, daß es sich bei der chemischen Reaktion um eine Crack-Reaktion von 1,2-Dichlorethan handelt.

14. Verfahren nach einem der Ansprüche 1 bis 12, dadurch gekennzeichnet, daß es sich bei der chemischen Reaktion um eine Crack-Reaktion eines Gemisches aus Kohlenwasserstoffen und Wasserdampf handelt.

15. Verfahren nach einem der Ansprüche 1 bis 14, dadurch gekennzeichnet, daß die transversale Vibration des Rohrs so erfolgt, daß mindestens ein Punkt des Rohrs mit einer Amplitude vibriert, die größer als das 10⁻⁴-fache des Innendurchmessers des Rohrs ist.

16. Verfahren nach einem der Ansprüche 1 bis 15, dadurch gekennzeichnet, daß die transversale Vibration des Rohrs mit einer Frequenz von 100 bis 1000 Hz erfolgt.

17. Verfahren nach einem der Ansprüche 1 bis 16, dadurch gekennzeichnet, daß die transversale Vibration des Rohrs bei einer der Eigenfrequenzen des Rohrs erfolgt.

18. Vorrichtung zur Herstellung von einem oder mehreren chemischen Produkten, bestehend aus einem Ofen, der eine Thermostrahlungswand umfaßt, die mit einer Heizeinrichtung versehen ist, die aus Brennern besteht und durch die mindestens ein Rohr verläuft, in dem ein oder mehr Reaktanten zirkulieren, dadurch gekennzeichnet, daß sie eine mechanische Vibrationsquelle, die mechanisch mit dem Rohr verbunden ist, oder eine Quelle für eine Schalldruckwelle, die außerhalb des Rohrs angeordnet ist und in im wesentlichen senkrechter Richtung zum Rohr ausgerichtet ist, umfaßt, so daß eine transversale Vibration auf mindestens einen Teil des Rohrs ausgeübt wird und dadurch eine Beschränkung von Abscheidungen auf der Innenwand des Rohrs herbeigeführt wird.

19. Vorrichtung nach Anspruch 18, dadurch gekennzeichnet, daß es sich bei dem Rohr um ein Crack-Rohr mit einem durchschnittlichen Innendurchmesser von 100 bis 250 mm handelt.

20. Vorrichtung nach Anspruch 18 oder 19, dadurch gekennzeichnet, daß die mechanische Vibrationsquelle außerhalb der Thermostrahlungswand angeordnet ist.

21. Vorrichtung nach einem der Ansprüche 18 bis 20, dadurch gekennzeichnet, daß es sich bei der mechanischen Vibrationsquelle um eine vibrierende Maschine handelt.

22. Vorrichtung nach Anspruch 18 oder 19, dadurch gekennzeichnet, daß die Druckwelle durch eine vibrierende Membran erzeugt wird.

23. Vorrichtung nach Anspruch 18 oder 19, dadurch gekennzeichnet, daß die Druckwelle durch einen oder mehrere Brenner der Thermostrahlungswand erzeugt wird.

24. Vorrichtung nach Anspruch 23, dadurch gekennzeichnet, daß der oder die Brenner mit einer Einrichtung ausgerüstet sind, um Pulsationen der Zufuhr des Brennstoffs zu den Brennern hervorzurufen.

25. Vorrichtung nach Anspruch 24, dadurch gekennzeichnet, daß es sich bei der Einrichtung zur Erzeugung von Pulsationen um eine Vorrichtung zum periodischen Verschließen des Ausgangs zur Versorgung der Brenner mit Brennstoff handelt.

26. Vorrichtung nach einem der Ansprüche 18 bis 25, dadurch gekennzeichnet, daß es sich beim Ofen um einen DampfCrack-Ofen handelt, der zum Cracken eines Gemisches von Kohlenwasserstoffen und Wasserdampf bestimmt ist.

27. Vorrichtung nach einem der Ansprüche 18 bis 25, dadurch gekennzeichnet, daß es sich beim Ofen um einen Ofen zum thermischen Cracken von 1,2-Dichlorethan handelt.
